# EUROPÄISCHE PATENTSCHRIFT

(11) **EP 0 528 284 B1**
(45) Veröffentlichungstag und Bekanntmachung des Hinweises auf die Patenterteilung: **23.10.1996**
(21) Anmeldenummer: 92113453.2
(22) Anmeldetag: 07.08.1992
(51) Int. Cl.: A61F 2/36

(54) **Hüftgelenkprothese**
Hip joint prosthesis
Prothèse de l'articulation de la hanche

(30) Priorität: 19.08.1991 CH 2429/91
(43) Veröffentlichungstag der Anmeldung: 24.02.1993
(73) Patentinhaber: Intraplant AG, CH-6330 Cham (CH)
(72) Erfinder: Imhof, Martin, CH-6343 Rotkreuz (CH); Graf Reinhard, A-8850 Murau (AT)
(74) Vertreter: Patentanwälte Schaad, Balass, Menzl & Partner AG

(56) Entgegenhaltungen:
- EP-A- 0 038 908
- EP-A- 0 058 745
- EP-A- 0 135 755
- EP-A- 0 310 566
- DE-A- 2 851 598
- DE-A- 3 829 361
- DE-C- 3 913 874
- FR-A- 2 618 667
- FR-A- 2 662 932
- US-A- 4 546 501

## Beschreibung

Die Erfindung betrifft eine Hüftgelenkprothese für die Verankerung im Femur gemäss Oberbegriff des Anspruches 1.

Aus der EP-A-0 038 908 ist eine Hüftgelenkprothese mit einem Schaft, der in einer von anterior nach posterior in Schaftrichtung verlaufenden Ebene S-förmig gekrümmt ist, bekannt. Dabei weist der Schaft im proximalen Bereich eine Krümmung mit anterior gelegenem Krümmungsmittelpunkt auf, während der Krümmungsmittelpunkt der Krümmung im distalen Bereich posterior gelegen ist. Im weiteren ist der Schaft dieser bekannten Hüftgelenkprothese noch in einer von lateral nach medial in Schaftrichtung verlaufenden Ebene in einer Richtung gekrümmt. Durch diese Ausbildung des Schaftes wird zwar ein gegenüber Hüftgelenkprothesen mit geradem Schaft besserer Sitz im Oberschenkelknochen erreicht, doch wird auf diese Weise eine immer noch ungenügende Anpassung des Schaftverlaufes an den natürlichen Verlauf der Knochenmarkhöhle erzielt.

Aus der FR-A-2,618,667 ist eine Hüftgelenkprothese der im Oberbegriff des Anspruches 1 genannten Art bekannt, deren Schaft im oberen Teil verwunden ist und im unteren Teil geradlinig verläuft. Auf diese Weise wird hinsichtlich Anpassung der Schaftform an den Verlauf der Markhöhle gegenüber Prothesen der in der vorerwähnten EP-A- 0 038 908 beschriebenen Art eine gewisse Verbesserung erzielt.

Der vorliegenden Erfindung liegt nun die Aufgabe zugrunde, eine Hüftgelenkprothese der eingangs genannten Art zu schaffen, die ohne aufwendige Knochenbearbeitung problemlos in die Markhöhle des Oberschenkelknochens eingeführt werden kann und vor allem eine noch bessere Anpassung des Verlaufs des Schaftes an die natürliche Ausbildung der Markhöhle ermöglicht.

Diese Aufgabe wird erfindungsgemäss durch die Merkmale des Anspruches 1 gelöst.

Durch das Verwinden des Schaftes über seine ganze Länge wird das Einführen der Hüftgelenkprothese in die Markhöhle erleichtert. Im weiteren wird eine grosse Auflagefläche zum Oberschenkelknochen geschaffen, wodurch die auf den Knochen wirkenden Druckkräfte pro Flächeneinheit möglichst klein gehalten werden können.

Ist der vorzugsweise einen ovalen Querschnitt aufweisende Schaft gemäss Anspruch 3 an seiner medialen und lateralen Schmalseite abgeflacht, wird infolge Keilwirkung ein stabiler Sitz im Oberschenkelknochen erhalten. Zudem ergibt sich so eine Sicherung gegen Verdrehung.

Bei einer weiteren bevorzugten Ausgestaltung gemäss Anspruch 5 sind an den Breitseiten des Schaftes zum distalen Ende hin schmäler werdende Rillen vorhanden, die herstellungstechnisch bedingt sind, aber einen besseren Halt der Prothese im Oberschenkelknochen sowie eine Sicherung gegen Verdrehung gewährleisten.

Vorzugsweise liegen die durch die Mitte der beiden Schmalseiten des Schafts verlaufende Vertikalebene und die Mittellinie des Halses nicht in einer Ebene, sondern schliessen einen Winkel miteinander ein, wie das im Anspruch 4 definiert ist. Der Hals mündet somit etwas schräg in den Schaft ein. Diese etwas schräge Anordnung des Halses stellt praktisch eine Fortsetzung der Verwindung des Schaftes dar, die sich auch über den Hals hinaus im gleichen Drehsinn erstreckt. Dadurch wird eine noch bessere Anpassung an die Anatomie des Oberschenkelknochens erreicht.

Eine Ausführungsform einer erfindungsgemässen Hüftgelenkprothese wird nachfolgend anhand der Zeichnung näher beschrieben. Es zeigen:
- Fig. 1: eine Seitenansicht der Prothese von der nach anterior zeigenden Seite gesehen;
- Fig. 2: verschiedene Querschnitte durch die Prothese gemäss Fig. 1 in verschiedenen Höhen;
- Fig. 3: eine Schmalseite einer Prothese für das rechte Hüftgelenk, von der lateralen Seite aus gesehen;
- Fig. 4: eine Draufsicht auf die Prothese gemäss Fig. 3;
- Fig. 5: eine Seitenansicht einer Prothese für das linke Hüftgelenk, von der lateralen Seite aus gesehen.

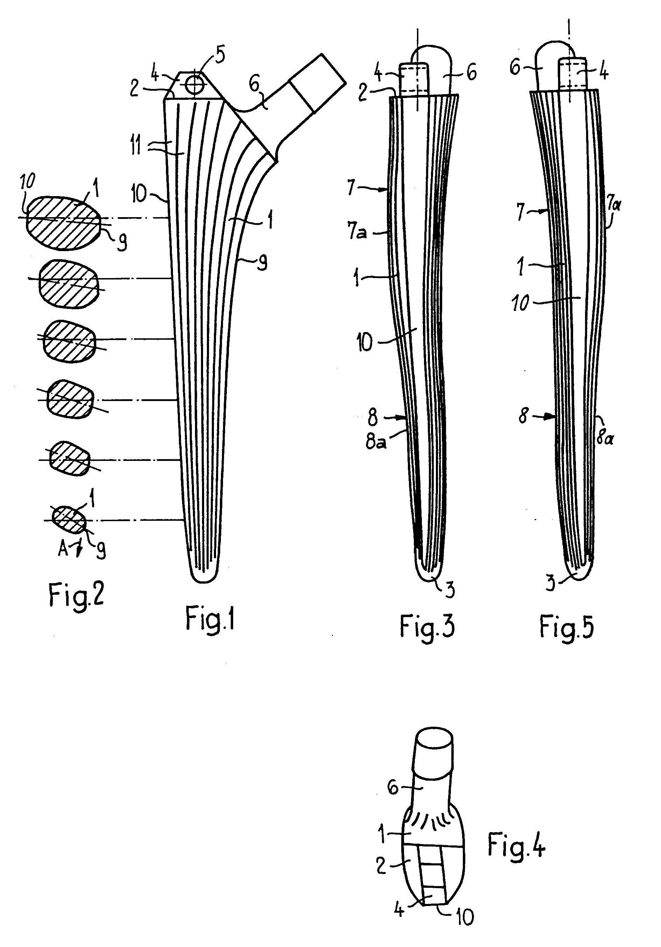

Die Hüftgelenkprothese weist einen Schaft 1 mit ovalem Querschnitt auf, der sich von einer oberen, ebenen Schulterfläche 2 zum distalen Ende 3 hin in der Breite und Dicke verjüngt, wie aus den verschiedenen Querschnitten gemäss Fig. 2 hervorgeht. Durch einen von der Schulterfläche 2 nach oben vorstehenden Vorsprung 4 erstreckt sich eine Bohrung 5 zum Einsetzen eines Werkzeugs, um die Prothese beim Einsetzen zu positionieren und wieder herausziehen zu können. Der an den Schaft 1 unter einem Winkel oben anschliessende Hals 6 dient zum Aufsetzen einer nicht dargestellten Gelenkkugel. Die Prothese gemäss Fig. 1 bis 4 ist für ein rechtes Hüftgelenk bestimmt, während die Prothese gemäss Fig. 5 für ein linkes Hüftgelenk bestimmt ist.

Aus Fig. 3 geht hervor, dass der Schaft 1 zwischen der oberen Schulterfläche 2 und dem distalen Ende 3 aneinander anschliessende Bereiche 7 und 8 aufweist, in denen er über die Breite unterschiedlich gekrümmt ausgebildet ist, wobei aneinander anschliessend gegensinning verlaufende Bögen 7a, 8a die Seitenkontur des Schafts 1 bilden und wobei der Schaft in der ersten von der Schulter 2 ausgehenden Krümmung, dass heisst in seinem Abschnitt 7, nach posterior konvex ausgebildet ist. Zusätzlich weist der Schaft 1 auch noch eine sich über seine ganze Länge erstreckende Verwindung im Sinne einer Schraubenlinie auf, wie aus den Querschnitten gemäss Fig. 2 hervorgeht, wobei die abgeflachte mediale Schmalseite 9 des Schafts 1 zum distalen Ende 3 hin zunehmend im Uhrzeigersinn oder nach anterior gerichtet gemäss Pfeil A in Fig. 2 verdreht ist. Mit dieser Formgebung ist eine optimale Anpassung an die Anatomie des Oberschenkelknochens erreicht.

Die für ein linkes Hüftgelenk bestimmte Prothese gemäss Fig. 5 ist hinsichtlich der anatomischen Anpassung an den Knochen genau gleich ausgebildet. Die Figuren 3 und 5 stellen in beiden Fällen eine Seitenansicht von der im wesentlichen geraden, ebenfalls abgeflachten lateralen Schmalseite 10 aus gesehen dar. Darum sind die Schäfte bezüglich der Krümmung quasi spiegelbildlich gleich dargestellt. Auch bei dem Schaft 1 der Prothese gemäss Fig. 5 ist der obere Abschnitt 7 nach prosterior konvex ausgebildet und der untere Abschnitt 8 entgegengesetzt gekrümmt. Die sich über die ganze Schaftlänge erstreckende Verwindung des Schafts ist in gleicher Weise derart ausgebildet, dass die mediale Schmalseite des Schafts zum distalen Ende 3 hin nach anterior verdreht ist. Von oben gesehen wäre dies eine Verwindung im Gegenuhrzeigersinn.

Aus Fig. 4 geht hervor, dass die Verwindung des Schafts 1 oder der leichte Drall sich in den Hals 6 fortsetzt, das heisst dieser Hals 6 liegt mit seiner Mittelachse nicht in einer durch die Mitte der beiden Schmalseiten 9, 10 des Schafts 1 verlaufenden Vertikalebene, sondern mündet etwas schräg unter einem spitzen Winkel in den Schaft 1.

Der Schaft 1 weist ferner zumindest an den beiden Breitseiten in Schaftlängsrichtung verlaufende und zum verjüngten Schaftende 3 hin schmäler werdende Rillen 11 auf, die eine sehr geringe Tiefe haben und bei der Herstellung des Schafts entstehen, die aber den Vorteil bieten, dass sie eine Sicherung gegen ein Verdrehen der eingesetzten Prothese darstellen. An der mit den Rillen 11 versehenen Aussenseite weist der Schaft 1 vorzugsweise eine Apatit-Beschichtung auf.

Die beschriebene Prothese eignet sich wegen der an die Anatomie des Oberschenkelknochens angepassten Schaftform ganz besonders für ein zementfreies Einsetzen in den Knochen.

## Patentansprüche

1. Hüftgelenkprothese für die Verankerung im Femur, mit einem blattartigen Schaft (1), der sich von der Schulter (2) zum unteren, distalen Ende (3) hin verjüngt und mit einem zum Aufstecken eines Gelenkkopfs bestimmten, unter einem Winkel in den Schaft (1) mündenden Hals (6), wobei der Schaft (1) am an die Schulter (2) anschliessenden oberen, proximalen Bereich (7) verwunden ist, dadurch gekennzeichnet, dass sich die Verwindung des Schaftes (1) in seiner Längsrichtung über seine ganze Länge erstreckt, wobei die mediale Schmalseite (9) des Schafts (1) zum distalen Ende (3) hin nach anterior verdreht ist und der Schaft (1) an den Breitseiten zwischen der Schulter (2) und dem distalen freien Ende (3) zwei aneinander anschliessende Bereiche (7,8) mit gegensinniger Krümmung aufweist, von denen der erste, von der Schulter (2) des Schaftes (1) ausgehende Bereich (7) an der posterioren Breitseite mit einer nach posterior konvex ausgebildeten Krümmung ausgebildet ist.

2. Hüftgelenkprothese nach Anspruch 1, dadurch gekennzeichnet, dass der Schaft (1) einen ovalen Querschnitt aufweist.

3. Hüftgelenkprothese nach Anspruch 1 oder 2, dadurch gekennzeichnet, dass der Schaft (1) an seiner medialen und lateralen Schmalseite (9,10) abgeflacht ist.

4. Hüftgelenkprothese nach einem der Ansprüche 1 bis 3, dadurch gekennzeichnet, dass der Hals (6) in Bezug auf eine durch die medialen und lateralen Schaftschmalseiten (9,10) verlaufende Vertikalmittelebene spitzwinklig in den Schaft (1) einmündet.

5. Hüftgelenkprothese nach einem der Ansprüche 1 bis 4, dadurch gekennzeichnet, dass die Oberfläche des Schafts (1) an den Breitseiten in Schaftlängsrichtung verlaufende, zum verjüngten distalen Schaftende (3) hin schmäler werdende Rillen (11) aufweist.

6. Hüftgelenkprothese nach einem der Ansprüche 1 bis 5, dadurch gekennzeichnet, dass die für ein linkes und ein rechtes Hüftgelenk bestimmten Prothesen in Bezug auf die Krümmung und die Verwindung des Schafts (1) spiegelbildlich gleich ausgebildet sind.

## Claims

1. A hip joint prosthesis for anchoring in the femur, with a leaf-like shank (2) which tapers from the shoulder (2) to the bottom distal end (3) and with a neck (6) fitted merging into the stem (1) at an angle and intended to be fitted on a joint head, the shank (1) being twisted at the upper proximal portion (7) which is adjacent the shoulder (2), characterised in that the twisting of the shank (1) extends in its longitudinal direction and over its entire length, the medial narrow side (9) of the shank (1) being twisted forwards towards the distal end (3) while the shank (1) has on the broad sides, between the shoulder (2) and the distal free end (3), two mutually adjacent portions (7, 8) of oppositely directed curvature, of which the first portion (7) starting from the shoulder (2) of the shank (1) is on the posterior broad side constructed with a curvature constructed convexly towards the rear.

2. A hip joint prosthesis according to claim 1, characterised in that the shank (1) has an oval cross-section.

3. A hip joint prosthesis according to claim 1 or 2, characterised in that the shank (1) is flattened on its medial and lateral narrow side (9, 10).

4. A hip joint prosthesis according to one of claims 1 to 3, characterised in that the neck (6) merges into the shank (1) at an acute angle in relation to a vertical central plane extending through the medial and lateral narrow sides (9, 10) of the shank.

5. A hip joint prosthesis according to one of claims 1 to 4, characterised in that the surface of the shank (1) has on the broad sides and extending in the longitudinal direction of the shank grooves (11) which become narrower towards the tapered distal end (3) of the shank.

6. A hip joint prosthesis according to one of claims 1 to 5, characterised in that the prostheses intended for a left hand and a right hand hip joint are identically constructed but are mirrored opposites with regard to the curvature and the twist of the shank (1).

## Revendications

1. Prothèse de l'articulation de la hanche destinée à être ancrée dans le fémur, comportant une tige du genre lame (1) qui s'amincit de l'épaulement (2) vers l'extrémité inférieure distale (3), et un col (6) se joignant sous un angle à la tige (1) et destiné à recevoir une tête d'articulation, la tige (1) étant tordue dans sa zone supérieure proximale (7) contiguë à l'épaulement (2), caractérisée par le fait que la torsion de la tige (1) s'étend dans la direction longitudinale de celle-ci sur toute sa longueur, la petite face médiale (9) de la tige (1) étant tordue vers l'avant vers l'extrémité distale (3) et la tige (1) présentant sur ses grandes faces entre l'épaulement (2) et l'extrémité libre distale (3) deux zones contiguës (7, 8) à courbure de sens contraire dont la première (7), partant de l'épaulement (2) de la tige (1), est pourvue d'une courbure convexe vers l'arrière sur la grande face postérieure.

2. Prothèse de l'articulation de la hanche selon la revendication 1, caractérisée par le fait que la tige (1) est de section ovale.

3. Prothèse de l'articulation de la hanche selon l'une des revendications 1 et 2, caractérisée par le fait que la tige (1) est aplatie sur sa petite face médiale (9) et sa petite face latérale (10).

4. Prothèse de l'articulation de la hanche selon l'une des revendications 1 à 3, caractérisée par le fait que le col (6) se joint à la tige (1) sous un angle aigu relativement à un plan médian vertical traversant les petites faces médiale et latérale (9, 10) de la tige.

5. Prothèse de l'articulation de la hanche selon l'une des revendications 1 à 4, caractérisée par le fait que la surface de la tige (1) présente des rainures (11) s'étendant dans la direction longitudinale de la tige sur les grandes faces et se rétrécissant vers son extrémité distale rétrécie (3).

6. Prothèse de l'articulation de la hanche selon l'une des revendications 1 à 5, caractérisée par le fait que les prothèses destinées à une articulation de la hanche gauche et une articulation de la hanche droite sont images l'une de l'autre dans un miroir en ce qui concerne la courbure et la torsion de la tige (1).
